(19) Europäisches Patentamt
European Patent Office
Office européen des brevets

(11) Publication number: **0 072 623**
**B1**

# (12) EUROPEAN PATENT SPECIFICATION

(45) Date of publication of patent specification: **05.06.85**

(51) Int. Cl.$^4$: **C 07 D 233/60,** A 61 K 31/415

(21) Application number: **82303656.1**

(22) Date of filing: **13.07.82**

## (54) Imidazole antifungal agents.

(30) Priority: **18.07.81 GB 8122246**

(43) Date of publication of application:
**23.02.83 Bulletin 83/08**

(45) Publication of the grant of the patent:
**05.06.85 Bulletin 85/23**

(84) Designated Contracting States:
**BE DE FR GB IT LU NL**

(56) References cited:
**EP-A-0 001 654**
**EP-A-0 019 190**
**EP-A-0 023 103**
**EP-A-0 040 345**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE DE FR IT LU NL**

(72) Inventor: **Gymer, Geoffrey Edward**
**19 Palmer Road**
**Wingham Canterbury (GB)**
Inventor: **Richardson, Kenneth**
**48 St. Stephens Hill**
**Canterbury Kent (GB)**

(74) Representative: **Moore, James William**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 072 623 B1

Courier Press, Leamington Spa, England.

**Description**

This invention relates to novel imidazole derivatives and in particular to certain 2-aryl-1-imidazolyl-8-piperazinylphenoxy-octan-2-ol derivatives which have antifungal activity and are useful in the treatment or prevention of fungal infections in animals, including humans.

Thus according to the invention there are provided compounds of the formula:

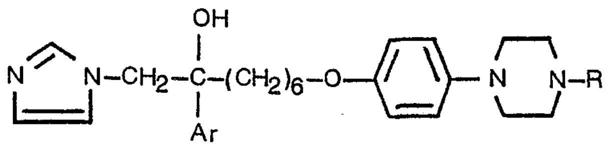

(I)

wherein R is hydrogen, $C_1$—$C_4$ alkyl, $C_3$—$C_7$ cycloalkyl, $CONH_2$, $CON(C_1—C_4\ alkyl)_2$, or substituted $C_1$—$C_4$ alkyl wherein the substituent is $N(C_1—C_4\ alkyl)_2$, one or two OH groups, $CONH_2$, $CON(C_1—C_4\ lower\ alkyl)_2$ or cycloalkyl;

Ar is phenyl optionally substituted by one or two halogen atoms, $C_1$—$C_4$ alkyl or $C_1$—$C_4$ alkoxy groups; and pharmaceutically acceptable acid addition salts thereof.

Alkyl groups containing 3 or 4 carbon atoms may be straight or branched-chain. Halogen means fluorine, chlorine, bromine or iodine. Ar is preferably a 2,4-dichlorophenyl group. R is preferably a $C_1$—$C_4$ alkyl group, especially an n-propyl or isopropyl group.

The invention also provides a pharmaceutical composition comprising a compound of the formula (I) or a pharmaceutically acceptable salt thereof, together with a pharmaceutically acceptable diluent or carrier.

The invention also provides a compound of the formula (I) or a pharmaceutically acceptable salt or pharmaceutical composition thereof for use in treating fungal infections (including prophylactic treatment) in animals including humans.

The compounds of the formula (I) may be obtained by a number of different processes. In one process according to the invention the compounds of the formula (I) are prepared by reacting a compound of the formula:

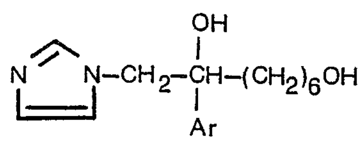

(II)

where Ar is as previously defined; with a compound of the formula:

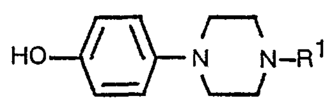

(III)

where $R^1$ is as defined for R or is a readily removable protecting group (e.g. acetyl); in the presence of triphenylphosphine and diethyl azodicarboxylate, and in the case where $R^1$ is a protecting group, removing the protecting group to give compounds of formula I wherein R is hydrogen, and optionally using a conventional alkylation reaction to obtain those compounds wherein R is alkyl, substituted alkyl or cycloalkyl or reacting with an alkali metal cyanate to obtain those compounds wherein R is $CONH_2$; and optionally forming a pharmaceutically acceptable acid addition salt of the product.

The reaction between the compounds of the formulae (II) and (III) is generally performed by dissolving the compounds in equimolar proportions, or using a slight excess (for example a 10% excess) of the phenol component (III), in a reaction-inert organic solvent. Tetrahydrofuran is a suitable solvent. The triphenylphosphine and diethyl azodiarboxylate reagents are then added, again generally in equimolar amounts, although further quantities can be added if desired to complete the reaction. The time taken for the reaction to go to completion naturally depends on the precise nature of the reactants and the temperature employed; we have found, however, that an overnight period at room temperature is generally sufficient. The reaction mixture is then worked up by conventional techniques. In practice this is generally done by extracting the basic product into dilute aqueous acid, washing with an organic solvent and basifying to precipitate the product, which may then be extracted into a water-immiscible organic solvent; evaporation gives the crude product. Further purification may be achieved, if desired, by conventional techniques, for example, by using column chromatography or by forming a salt of the product (for example to oxalate salt) which may be recrystallised from a suitable solvent.

In the case of the compounds of formula I wherein R is hydrogen, the reaction is performed using an intermediate of formula (III) wherein the group $R^1$ is a readily removable protecting group. A particularly suitable example is the acetyl group. The group is then removed as a further step in the reaction. In the case

of the acetyl group this is readily achieved by heating the N-acetyl derivative with hydrochloric acid, a period of 2 or 3 hours at 70°C being sufficient to ensure complete reaction.

The compound of formula I wherein R is H can also be employed as a convenient starting material for several of the other compounds of the invention by using conventional chemical transformation reactions. Thus reductive alkylation with an appropriate aldehyde or ketone, or substituted aldehyde or ketone, suitably in the presence of sodium cyanoborohydride leads to the compounds of formula (I) wherein R is a lower alkyl, substituted lower alkyl or cycloalkyl group. Similarly reaction with an alkali metal cyanate, for example, sodium cyanate, suitably in glacial acetic acid yields the compounds of formula I wherein R is a carbamoyl group. These reactions are entirely conventional and methods and conditions for their performance will be well known to those skilled in the art.

The starting materials of formula (II) are prepared from the known 2-(1-imidazolyl)acetophenone derivatives according to the following reaction scheme:

REACTION SCHEME

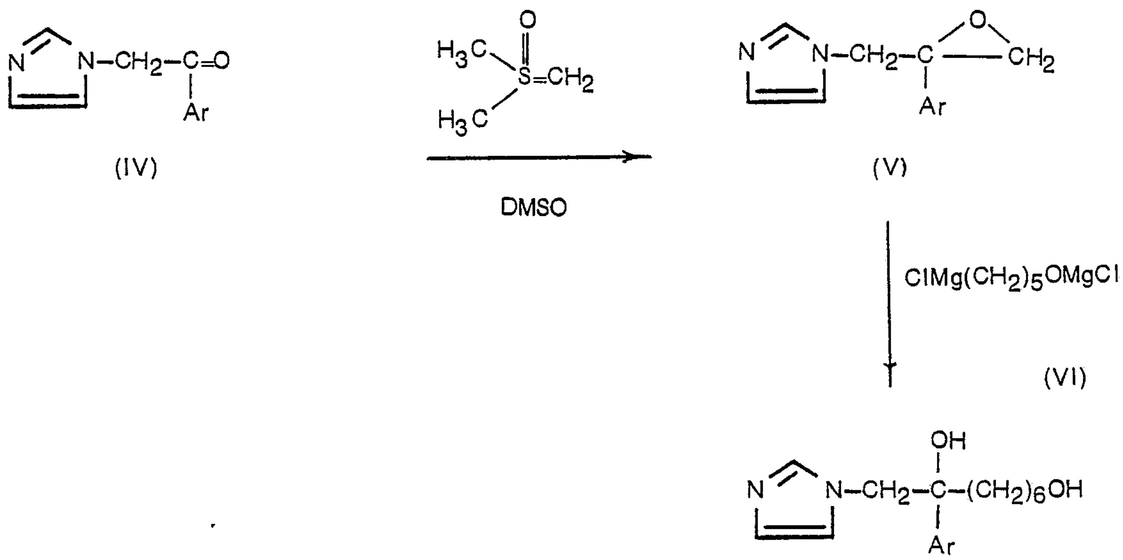


(IV) (V) (VI) (II)

The oxirane (V) may be prepared from a ketone of the formula (IV) by reacting with the methylide prepared from trimethyloxosulphonium iodide and sodium hydride. This reaction is generally achieved by adding powdered trimethylsulphoxonium iodide to a suspension of sodium hydride in dimethylsulphoxide, and, after a few minutes, the ketone (IV) is added, in equimolar amount. The reaction mixture may be warmed to accelerate the reaction and after several hours at 50—80°C the product is isolated in a conventional manner, for example, by pouring into water, extracting with an organic solvent and recrystallisation. The reaction of the oxirane (V) with the Grignard reagent (VI) is performed in a conventional manner, for example by adding a solution of oxirane in an inert organic solvent to the freshly prepared Grignard reagent. The reaction is allowed to proceed at room temperature for some hours, generally overnight. The product is then recovered in a conventional manner, for example by adding aqueous ammonium chloride, separating the organic phase and evaporating the solvent. The crude product may be further purified, if necessary, by recrystallisation or by column chromatography.

The phenols of formula (III) are known compounds or they may be simply prepared by acylating or alkylating the unsubstituted piperazine compound where $R^1 = H$ using conventional techniques.

Pharmaceutically acceptable acid addition salts of the compounds of the invention are those formed from acids which form non-toxic acid addition salts containing pharmaceutically-acceptable anions, such as the hydrochloride, hydrobromide, sulphate or bisulphate, phosphate or acid phosphate, acetate, maleate, furmarate, lactate, tartrate, citrate, gluconate, succinate, oxalate, and *p*-toluene-sulphonate salts. The salts may be obtained by conventional procedures, e.g. by mixing solutions containing equimolar amounts of the free base and desired acid, and the required salt is collected by filtration, if insoluble, or by evaporation of the solvent.

The compounds of the formula (I) and their pharmaceutically acceptable salts are anti-fungal agents, useful in combating fungal infections in animals, including humans. For example they are useful in treating tropical fungal infections in man caused by, among other organisms, species of *Candida, Trichophyton, Microsporum,* or *Epidermophyton,* or in mucosal infections caused by *Candida albicans* (e.g. thrush and

vaginal candidiasis). They may also be used systemically in the treatment of systemic fungal infections caused by, for example, *Candida albicans, Cryptococcus neoformans, Aspergillus fumigatus, Coccidioides, Paracoccidioides, Histoplasma,* or *Blastomyces.* In addition because of their improved fungicidal activity the compounds are highly active in immune suppressed animals. Thus the compounds could be of particular value in the treatment and prevention of fungal infections in immune suppressed patients, such as in patients undergoing cancer therapy or after organ transplant operations.

The *in vitro* evaluation of the anti-fungal activity of the compounds can be performed by determining the minimum inhibitory concentration (m.i.c.) of the test compounds in a suitable medium at which growth of the particular micro-organism fails to occur. In practice, a series of agar plates, each having the test compound incorporated at a particular concentration are inoculated with a standard culture of, for example, *Candida albicans* and each plate is then incubated for 48 hours at 37°C. The plates are then examined for the presence or abesence of growth of the fungus and the appropriate m.i.c. value is noted. Other micro-organisms used in such tests can include *Cryptococcus neoformans, Aspergillus fumigatus, Trichophyton* spp; *Microsporum* spp; *Epidermophyton floccosum, Coccidioides immitus,* and *Torulopsis glabrata.*

The *in vivo* evaluation of the compounds can be carried out at a series of dose levels by intraperitoneal or intravenous injection or by oral administration, to mice which are inoculated with a strain of *Candida albicans.* Activity is based on the survival of a treated group of mice after the death of an untreated group of mice following 48 hours observation. The dose level at which the compound provides 50% protection against the lethal effect of the infection is noted.

For human use, the anti-fungal compounds of the formula (I) can be administered alone, but will generally by administered in admixture with a pharmaceutical carrier selected with regard to the intended route of administration and standard pharmaceutical practice. For example, they may be administered orally in the form of tablets contining such excipients as starch or lactose, or in capsules either alone or in admixture with excipients, or in the form of elixers or suspensions containing flavouring or colouring agents. They may be injected parenterally, for example, intravenously, intramuscularly or subcutaneously. For paarenteral administration, they are best used in the form of a sterile aqueous solution which may contain other substances, for example, enough salts or glucose to make the solution isotonic.

For oral and parenteral administration to human patients, it is expected that the daily dosage level of the anti-fungal compounds of the formula (I) will be from 0.1 to 10 mg/kg (in divided doses) when administered by either the oral or parenteral route. Thus tablets or capsules of the compounds can be expected to contain from 5 mg to 0.5 g of active compound for administration singly or two or more at a time as appropriate. The physician in any event will determine the actual dosage which will be most suitable for an individual patient and it will vary with the age, weight and response of the particular patient. The above dosages are exemplary of the average case. There can, of course, be individual instances where higher or lower dosage ranges are merited, and such are within the scope of this invention.

Alternatively, the anti-fungal compounds of formula (I) may be administered in the form of a suppository or pessary, or they may be applied topically in the form of a cream, ointment or dusting powder. For example, they may be incorporated into a cream consisting of an aqueous emulsion of polyethylene glycols or liquid paraffin; or they may be incorporated, at a concentration between 1 and 10%, into an ointment consisting of a white wax or white soft paraffin base together with such stabilizers and perservatives as may be required.

The following Examples illustrate the preparation of the novel compounds of the formula (I):—

## Example 1

2-(2,4-Dichlorophenyl)-1-(imidazol-1-yl)-8-(4-piperazin-1-ylphenoxy)octan-2-ol;

(i) Triphenylphosphine (2.56 g) and diethyl azodicarboxylate (1.71 g) were added to a stirred solution of 2-(2,4-dichlorophenyl)-1-(1-imidazolyl)octan-2,8-diol (3.5 g) and 1-acetyl-4-(4-hydroxyphenyl)piperazine (2.16 g) in dry tetrahydrofuran (70 ml) under nitrogen: stirring was continued at room temperature for 20 hours; further triphenylphosphine (1.28 g) and diethyl azodicarboxylate (0.85 g) were added, and the stirring continued for a further 20 hours. The mixture was then poured into methylene chloride (100 ml) and extracted with N-hydrochloric acid (3 × 50 ml). The combined acidic extracts were washed with methylene chloride (3 × 30 ml) and basified to pH10 by the addition of 2N sodium hydroxide with cooling in ice. The solution was extracted with methylene chloride (3 × 50 ml), and the extracts combined, dried over $MgSO_4$ and evaporated to yield a gum (4.87 g). Chromatography on silica eluting with methylene chloride (150 ml) followed by a mixture of methylene chloride, isopropyl alcohol (2%) and concentrated ammonium hydroxide (0.2%) gave a gum which was taken up in ethyl acetate and converted to the oxalate salt by the dropwise addition of a saturated solution of anhydrous oxalic acid in diethyl ether until no further precipitation occurred. The resulting precipitate was collected by filtration, washed with a little diethyl ether and dried under vacuum to give the N-acetyl derivative of the title compound as its dioxalate salt (6.7 g, 90%) m.p. 75—78°C. Rf (silica, methylene chloride: isopropyl alcohol: ammonium hydroxide, 90:10:1) 0.3 m/e 558.

(ii) The product from (i) (3.5 g) was dissolved in 5N hydrochloric acid (40 ml) and the solution heated at 70°C for 2 hours. After cooling, 2N sodium hydroxide solution was added to pH 9.0 and the solution was then evaporated under vacuum to yield a gum. The residue was extracted with chloroform (3 × 50 ml) and

with hot ethyl acetate (3 × 50 ml). The extracts were dried over $MgSO_4$ and evaporated to give the title compound as a foam (1.85 g, 75%). Rf (silica:methylene chloride:isopropylalcohol:ammonium hydroxide, 70:30:1) 0.25. A sample was converted to the dioxalate salt as previously described.

Found (dioxalate salt): C, 51.73; H, 5.40; N, 8.04.
$C_{27}H_{34}Cl_2N_4O_2 \cdot 2C_2H_2O_4$ requires: C, 53.38; H, 5.40; N, 8.04%.

Example 2

2-(2,4-Dichlorophenyl)-1-(imidazol-1-yl)-8-[4-(4-carbamoylmethylpiperazin-1-yl)phenoxy]octan-2-ol

Triphenylphosphine (0.393 g) and ethyl azodicarboxylate (0.261 g) were added to a stirred solution of 2-(2,4-dichlorophenyl)-1-(1-imidazolyl)-octan-2,8-diol (0.357 g) and 1-carbamoylmethyl-4-(4-hydroxyphenyl)piperazine (0.258 g) in dry tetrahydrofuran. Stirring was continued at room temperature for 19 hours and the reaction mixture was then added to methylene chloride (35 ml) and extracted with N-hydrochloric acid (3 × 20 ml). The combined acidic extracts were washed with methylene chloride (3 × 15 ml), basified by the addition of 2N sodium hydroxide solution with cooling in ice, and extracted with methylene chloride (3 × 20 ml). The combined organic extracts were washed with saturated brine, dried over $MgSO_4$ and evaporated to give the title compound as a gum which solidified on scratching under diisopropyl ether (0.206 g, 35%). A sample was converted to the dioxalate salt as previously described. m.p. 113—116°C. Rf (silica, dichloromethane:isopropyl alcohol:ammonium hydroxide 70:30:1) 0.62.

Found (dioxalate salt): C, 51.75; H, 5.77; N, 9.06.
$C_{29}H_{37}Cl_2N_5O_3 \cdot 2C_2H_2O_4$ requires: C, 51.30; H, 5.61; N, 9.06%. m/e 573.

Example 3

2-(2,4-Dichlorophenyl)-1-(imidazol-1-yl)-8-[4-(4-isopropylpiperazin-1-yl)phenoxy] octan-2-ol

The procedure of Example 2 was followed but using 1-isopropyl-4-(4-hydroxyphenyl)piperazine instead of the 1-carbamoylmethyl derivative. The crude product was recrystallized from a mixture of ethyl acetate and diisopropyl ether to give the title compound (2.21 g; 38%) as a cream solid. m.p. 143—145°C. Rf (silica, diethyl ether:ethanol:ammonium hydroxide, 90:10:1) 0.61; m/e 558

Found: C, 63.99; H, 7.44; N, 10.13.
$C_{30}H_{40}Cl_2N_4O_2$ requires: C, 64.40; H, 7.20; N, 10.01%.

Example 4

2-(2,4-Dichlorophenyl)-1-(imidazol-1-yl)-8-[4-(4-propylpiperazin-1-yl)phenoxy]octan-2-ol

Propionaldehyde (43.5 mg) and sodium cyanoborohydride (47.5 mg) were added to a solution of 2-(2,4-dichlorophenyl)-1-(imidazol-1-yl)-8-(4-piperazin-1-ylphenoxy)octan-2-ol (262 mg) in ethanol (5 ml). The pH of the solution was adjusted to 6 by the dropwise addition of 2N hydrochloric acid and tetrahydrofuran (5 ml) and water (2 ml) were then added to give a slighly cloudy solution which was allowed to stir at room temperature for two hours. Further propionaldehyde (43.5 mg) and sodium cyanoborohydride (47.5 mg) were added and the mixture was allowed to stir at room temperature for a further 48 hours. Water (50 ml) and sodium chloride (5 g) were added and the solution extracted with methylene chloride (3 × 50 ml). The combined organic extracts were washed with brine, dried over $MgSO_4$ and evaporated to yield a gum. Chromatography on silica (10 g) eluting with methylene chloride containing increasing amounts of methanol gave the title compound as a glass (110 mg, 27%). m.p. 113—117°C, Rf (silica, diethylether; ethanol; ammonium hydroxide, 90:10:1) 0.7. m/e 558.

Found: C, 64.5; H, 7.18; N, 10.2.
$C_{30}H_{40}Cl_2N_4O_2$ requires: C, 64.5; H, 7.23; N, 10.0%.

Example 5—10

The following compounds were prepared by the general procedure of Example 4, starting with the appropriate aldehyde or ketone. The compounds are of the formula I wherein Ar is 2,4-dichlorophenyl and R is as shown. The Rf values are for thin layer chromatography on silica, using the solvent system indicated below.

| Example | R | Rf | Analysis (Theoretical in brackets) C | H | N |
|---|---|---|---|---|---|
| 5 | $—CH_2CH_3$ | 0.55[1] | Not obtained; m/e 545 | | |
| 6 | 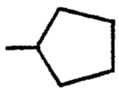 | 0.75[2] | 65.2 (65.7 | 7.21 7.24 | 9.59 9.58) |
| 7 | $—CH_2CH_2N(CH_3)_2$ | 0.74[3] | 47.32 (49.37 | 5.26 5.41 | 7.19 7.38)[5] |
| 8 | $—CH_2CHOHCH_2OH$ | 0.3[4] | 49.91 (50.18 | 5.62 5.38 | 6.62 6.50)[6] |
| 9 | $—CH(CH_2OH)_2$ | 0.5[4] | 52.15 (51.71 | 5.66 5.87 | 6.67 7.09)[7] |

1. Methylene chloride, isopropanol, ammonium hydroxide 85:15:1
2. Diethylether, ethanol, ammonium hydroxide 90:10:1
3. Chloroform, methanol, ammonium hydroxide 80:20:5
4. Methylene chloride, isopropanol, ammonium hydroxide 70:30:1
5. Tetraoxalate
6. Trioxalate
7. Dioxalate, hydrate.

### Example 10

2-(2,4-Dichlorophenyl)-1-(imidazol-1-yl)-8-[4-(4-carbamoylpiperazin-1-yl)phenoxy]octan-2-ol dioxalate monohydrate

Glacial acetic acid (1 ml) and sodium cyanate (0.15 g) were added to a solution of 2-(2,4-dichlorophenyl)-1-(imidazol-1-yl)-8-(4-piperazin-1-yl-phenoxy)octan-2-ol (0.3 g) in acetone (5 ml) at room temperature. After 40 minutes the solution was basified with 5N sodium hydroxide solution and extracted with ethyl acetate (3 × 20 ml). The combined extracts were washed with water, dried over $MgSO_4$ and evaporated to yield a gum. Chromatography on silica eluting with methylene chloride (200 ml) containing isopropyl alcohol (10 ml) and concentrated ammonium hydroxide (1 ml) followed by methylene chloride (200 ml) containing isopropyl alcohol (20 ml) and concentrated ammonium hydroxide (1 ml) gave the desired product which was converted to its dioxalate salt as previously described. (0.22 g, 50%). m.p. 99—102°C. Rf (silica, methylene chloride, isopropyl alcohol, ammonium hydroxide 70:30:1) 0.45. m/e 516 ($M—CONH_2$).

Found (dioxalate salt, hydrate): C, 50.66; H, 5.41; N, 8.81.
$C_{28}H_{35}Cl_2N_5O_3 \cdot 2C_2O_4H_2 \cdot H_2O$ requires: C, 50.66; H, 5.44; N, 9.23%.

### Example 11

The following illustrate pharmaceutical compositions for the treatment of fungal infections:

(1) *Capsule:* 71 parts by weight of the compound of Example 3 are granulated with 3 parts maize starch and 22 parts lactose and then a further 3 parts of maize starch and 1 part magnesium stearate are added. The mixture is regranulated and filled into hard gelatin capsules.

(2) *Cream:* 2 parts by weight of the compound of Example 3 are dissolved in 10 parts of propylene glycol and mixed into 88 parts of a vanishing cream base.

(3) *Pessary:* 2 parts by weight of the compound of Example 3 are suspended in 98 parts of a warm liquified suppository base which is poured into moulds and allowed to solidify.

### Preparation 1

2-(2,4-Dichlorophenyl)-2-(1-imidazolylmethyl)oxirane

Sodium hydride (50% dispersion in oil, 5.0 g, 0.11 mole) was washed with petrol to remove the oil, and dry dimethylsulphoxide (150 ml added. The mixture was stirred under dry nitrogen at room temperature while finely ground trimethylsulphoxonium iodide (22.0 g, 0.1 mole) was added in portions over a period of 20 minutes. After a further 10 minutes a solution of 2′,4′-dichloro-2-(1-imidazolyl) acetophenone (25.5 g, 0.1 mole) in dry dimethylsulphoxide (100 ml) was added over 20 minutes. The reaction mixture was heated at 65°C for 3 hours, cooled to room temperature and poured into a mixture of ice and water (1 ml). The product was extracted with diethyl ether (3 × 500 ml) and the combined ether extracts were washed with

water (2 × 250 ml), dried over $MgSO_4$ and evaporated to yield an oil. The oil was extracted with petroleum ether (b.p. 60—80°C, 5 × 250 ml) and the solvent evaporated. The resulting pale gum was crystallised from cyclohexane to give 2-(2,4-dichlorophenyl)-2-(1-imidazolylmethyl)oxirane (11.2 g, 42%) as colourless crystals, m.p. 84—85°C.

| | | | |
|---|---|---|---|
| Found (oxalate salt): | C, 44.06; | H, 3.52; | N, 6.74. |
| $C_{12}H_{10}Cl_2N_2O \cdot 1\frac{1}{2} \cdot C_2H_2O_4$ requires: | C, 44.57; | H, 3.24; | N, 6.93%. |

Preparation 2

2-(2,4-Dichlorophenyl)-1-(imidazol-1-yl)octan-2,8-diol

Dry tetrahydrofuran (50 ml) was added to magnesium turnings (2.4 g) and a solution of 2-chloropropane (7.96 g) in tetrahydrofuran (50 ml) was added over 10 minutes at such a rate as to maintain a gentle reflux, the reaction being initiated with a little methyl iodide and gentle warming. The resulting solution was transferred under nitrogen to a cooled solution of 5-chloropentan-1-ol (9.76 g) in tetrahydrofuran (200 ml) at −20°C. After stirring for 15 minutes magnesium turnings (7.2 g) were added and the reaction mixture was allowed to warm to room temperature. Sodium bis(2-methoxyethoxy) aluminium hydride (20 drops, 70% in benzene) was added and the mixture heated under reflux for 1 hour during which time 1,2-dibromoethane (0.6 ml) was added in three equal portions. Heating was continued for a further 1½ hours and the mixture was then cooled, diluted by the addition of dry tetrahydrofuran (100 ml) and the slurry added under nitrogen to a cooled mixture of cuprous iodide (1.52 g) in tetrahydrofuran (100 ml) at −30°C, the residue being washed in with further dry tetrahydrofuran (2 × 100 ml). A solution of 2-(2,4-dichlorophenyl)-2-(1-imidazolylmethyl)oxirane (5.3 g) in tetrahydrofuran (50 ml) was added with stirring over a period of 10 minutes, the temperature of the solution being maintained at −30°C. Stirring was continued overnight at room temperature and the resulting solution was diluted with aqueous ammonium chloride (200 ml). The solution was extracted with ethyl acetate (3 × 200 ml) and the combined organic extracts were washed with dilute ammonium hydroxide (3 × 200 ml), and saturated brine (3 × 200 ml), dried over $MgSO_4$ and evaporated to yield an oil. Chromatography on silica gel eluting with methylene chloride containing an increasing proportion of isopropyl alcohol containing 10% concentrated ammonium hydroxide gave the desired product (4.45 g, 63%), as a cream solid. Rf (silica, methylene chloride:isopropyl alcohol:ammonium hydroxide 90:10:1) 0.15. m.p. 74—76°C.

| | | | |
|---|---|---|---|
| Found: | C, 57.04; | H, 6.20; | N, 8.01. |
| $C_{17}H_{22}Cl_2N_2O_2$ requires: | C, 57.14; | H, 6.21; | N, 7.84%. |

Preparation 3

1-Carbamoylmethyl-4-(4-hydroxyphenyl)piperazine

Chloroacetamide (0.93 g) was added to a solution of N-(4-hydroxyphenyl)piperazine (1.78 g), in N,N-dimethylformamide (15 ml) and the mixture heated at 60—65°C for 18 hours. the solvent was removed under vacuum and the dark oily residue dissolved in methanol and chromatographed on silica eluting with methylene chloride containing increasing proportions of ispropyl alcohol and concentrated ammonium hydroxide. Evaporation of the relevant fractions gave the desired product as (0.33 g, 14%). Rf (silica, methylene chloride, isopropyl alcohol, ammonium hydroxide 90:10:1) 0.3.

| | | | |
|---|---|---|---|
| Found: | C, 60.80; | H, 7.23; | N, 17,75. |
| $C_{12}H_{17}N_3O_3$ requires: | C, 61.26; | H, 7.28; | N, 17.86%. |

Preparation 4

1-Isopropyl-4-(4-hydroxyphenyl)piperazine

Sodium cyanoborohydride (3.78 g) was added to a stirred solution of N-(4-hydroxyphenyl)piperazine (5.35 g) and acetone (8.7 g) in a mixture of methanol (100 ml) and water (20 ml). The pH of the resulting solution was adjusted to 7.5 by the addition of a few drops of N-hydrochloric acid. Stirring was continued for 19 hours when further acetone (8.7 g) and sodium cyanoborohydride (1.89 g) were added and the pH readjusted to 7.5. After a further 8 hours water (100 ml) was added to the reaction mixture. The precipitated solid was collected by filtration, dried under vacuum over phosphorus pentoxide and recrystallized from a mixture of ethanol and methanol to give the desired product as a crystalline solid (4.78 g, 72%). m.p. 244—246°C.

| | | | |
|---|---|---|---|
| Found: | C, 70.49; | H, 9.09; | N, 12,86. |
| $C_{13}H_{20}N_2O$ requires: | C, 70.86; | H, 9.15; | N, 12.78%. |

**Claims**

1. A compound of the formula:

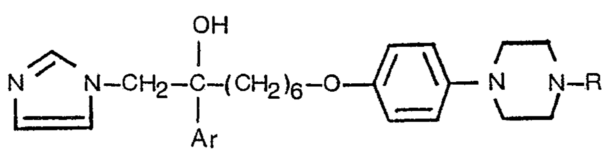

(I)

wherein R is hydrogen, $C_1$—$C_4$ alkyl, $C_3$—$C_7$ cycloalkyl, $CONH_2$, CON($C_1$—$C_4$ alkyl)$_2$, or substituted $C_1$—$C_4$ alkyl wherein the substituent is N($C_1$—$C_4$ alkyl)$_2$, one or two OH groups, $CONH_2$, CON($C_1$—$C_4$ lower alkyl)$_2$ or cycloalkyl;

Ar is phenyl optionally substituted by one or two halogen atoms, $C_1$—$C_4$ alkyl or $C_1$—$C_4$ alkoxy groups; and pharmaceutically acceptable acid addition salts thereof.

2. A compound as claimed in claim 1 wherein Ar is 2,4-dichlorophenyl.

3. A compound as claimed in claim 1 or claim 2 wherein R is a $C_1$—$C_4$ alkyl group.

4. A compound as claimed in claim 3 wherein R is an n-propyl or an isopropyl group.

5. A process for preparing a compound of the formula I as claimed in claim 1 which comprises reacing a compound of the formula

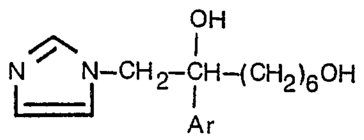
(II)

where Ar is as defined in claim 1 with a compound of the formula:

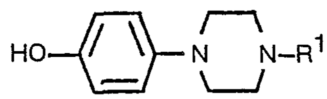
(III)

where $R^1$ is as defined in claim 1 for R or is a readily removable protecting group, in the presence of triphenylphosphine and diethyl azodicarboxylate, and in the case where $R^1$ is a protecting group, removing the protecting group to give compounds of formula I wherein R is hydrogen, and optionally using a conventional alkalation reaction to obtain those compounds wherein R is alkyl, substituted alkyl or cycloalkyl; or reacting with an alkali metal cyanate to obtain those compounds wherein R is $CONH_2$; and optionally forming a pharmaceutically acceptable acid addition salt of the product.

6. A pharmaceutical composition comprising a compound of the formula (I) as claimed in any one of claims 1 to 4, or a pharmaceutically acceptable acid addition salt thereof, together with a pharmaceutically acceptable diluent or carrier.

## Patentansprüche

1. Verbindung der Formel

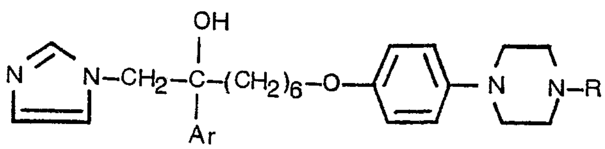
(I)

in welcher R Wasserstoff, $C_1$—$C_4$-Alkyl, $C_3$—$C_7$-Cycloalkyl, $CONH_2$, CON($C_1$—$C_4$-Alkyl)$_2$ oder substituiertes $C_1$—$C_4$-Alkyl, wobei der Substituent N($C_1$—$C_4$-Alkyl)$_2$, ein oder zwei OH Gruppen, $CONH_2$, CON($C_1$—$C_4$-Niederalkyl)$_2$ oder Cycloalkyl ist;

Ar Phenyl gegebenenfalls substituiert durch ein oder zwei Halogenatome, $C_1$—$C_4$-Alkyl oder $C_1$—$C_4$-Alkoxygruppen;

bedeuten und pharmazeutisch annehmbare Säureadditionssalze davon.

2. Verbindung gemäß Anspruch 1, worin Ar 2,4-Dichlorophenyl ist.

3. Verbindung gemäß Anspruch 1 oder 2, worin R eine $C_1$—$C_4$-Alkylgruppe ist.

4. Verbindung gemäß Anspruch 3, worin R eine n-Propyl oder eine Isopropyl-Gruppe ist.

5. Verfahren zur Herstellung einer Verbindung der Formel I gemäß Anspruch 1, wobei eine Verbindung der Formel

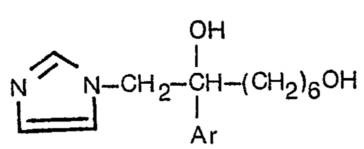
(II)

in welcher Ar die in Anspruch 1 angegebene Bedeutung besitzt, mit einer Verbindung der Formel

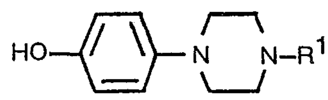

(III)

in welcher $R^1$ die in Anspruch 1 für R angegebene Bedeutung besitzt oder eine leicht entfernbare Schutzgruppe darstellt, in Anwesenheit von Triphenylphosphin und Diethylazodicarboxylat umgesetzt wird, und im Falle, daß $R^1$ eine Schutzgruppe darstellt, die Schutzgruppe entfernt wird zur Herstellung von Verbindungen der Formel I, worin R Wasserstoff darstellt, und gegebenenfalls unter Verwendung von konventionellen Alkylierungsreaktionen solche Verbindungen hergestellt werden, worin R Alkyl, substituiertes Alkyl oder Cycloalkyl bedeutet; oder durch Umsetzung mit einem Alkalimetallcyanat solche Verbindungen hergestellt werden, worin R $CONH_2$ bedeutet; und gegebenenfalls ein pharmazeutisch annehmbares Säureadditionssalz des Produktes gebildet wird.

6. Eine pharmazeutische Zusammensetzung enthaltend eine Verbindung der Formel I gemäß einem der Ansprüche 1 bis 4 oder ein pharmazeutisch annehmbares Säureadditionssalz davon zusammen mit einem pharmazeutisch annehmbaren Verdünnungsmittel oder Träger.

**Revendications**

1. Composé de formule:

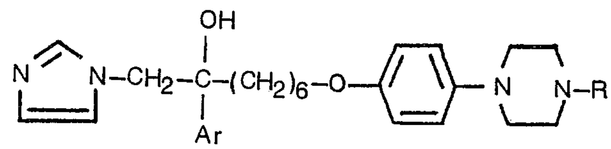

(I)

dans laquelle R est l'hydrogène, un groupe alkyle en $C_1$ à $C_4$, cycloalkyle en $C_3$ à $C_7$, $CONH_2$ CON(alkyle en $C_1$ à $C_4)_2$, ou alkyle en $C_1$ à $C_4$ substitué dont le substituant est un radical N(alkyle en $C_1$ à $C_4)_2$, un ou deux groupes OH, $CONH_2$, CON(alkyle inférieur en $C_1$ à $C_4)_2$ ou cycloalkyle;

Ar est un groupe phényle éventuellement substitué par un ou deux atomes d'halogènes, groupes alkyle en $C_1$ à $C_4$ ou groupes alkoxy en $C_1$ à $C_4$;

et ses sels d'addition d'acides pharmaceutiquement acceptables.

2. Composé suivant la revendication 1, dans lequel Ar est le groupe 2,4-dichlorophényle.

3. Composé suivant la revendication 1 ou la revendication 2, dans lequel R est un groupe alkyle en $C_1$ à $C_4$.

4. Composé suivant la revendication 3, dans lequel R est un groupe n-propyle ou un groupe isopropyle.

5. Procédé de préparation d'un composé de formule I suivant la revendication 1, qui consiste à faire réagir un composé de formule

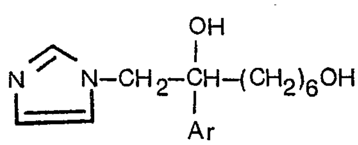

(II)

dans laquelle Ar a la définition donnée dans la revendication 1, avec un composé de formule:

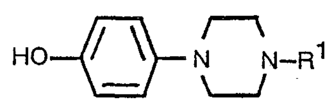

(III)

dans laquelle $R^1$ a la définition donnée dans la revendication 1 pour R ou est un groupe protecteur facile à éliminer, en présence de triphénylphosphine et d'azodicarboxylate de diéthyle, et au cas où $R^1$ est un groupe protecteur, à éliminer le groupe protecteur pour obtenir des composés de formule I dans laquelle R est l'hydrogène, et le cas échéant, à utiliser une réaction classique d'alkylation pour obtenir des composés dans lesquels R est un groupe alkyle, alkyle substitué ou cycloalkyle; ou à le faire réagir avec un cyanate de métal alcalin pour obtenir des composés dans lesquels R est un groupe $CONH_2$; et le cas échéant, à former un sel d'addition d'acide pharmaceutiquement acceptable du produit.

6. Composition pharmaceutique, comprenant un composé de formule I suivant l'une quelconque des revendications 1 à 4 ou un sel d'addition d'acide pharmaceutiquement acceptable de ce composé, en association avec un diluant ou support acceptable du point de vue pharmaceutique.